(19) Europäisches Patentamt
European Patent Office
Office européen des brevets

(11) **EP 2 090 182 A1**

(12) **EUROPEAN PATENT APPLICATION**

(43) Date of publication:
**19.08.2009 Bulletin 2009/34**

(51) Int Cl.:
*A23L 1/275* (2006.01)   *C12P 23/00* (2006.01)
*A61K 8/97* (2006.01)   *A61K 31/122* (2006.01)
*A61K 36/05* (2006.01)

(21) Application number: **09001218.8**

(22) Date of filing: **29.01.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **31.01.2008 JP 2008021589**
**19.11.2008 JP 2008295537**

(71) Applicant: **Yamaha Hatsudoki Kabushiki Kaisha Iwata-shi, Shizuoka 438-8501 (JP)**

(72) Inventors:
• **Suzuki, Rie**
**Iwata-shi**
**Shizuoka 438-8501 (JP)**

• **Sugiyama, Hiroyuki**
**Iwata-shi**
**Shizuoka 438-8501 (JP)**
• **Saiki, Asako**
**Iwata-shi**
**Shizuoka 438-8501 (JP)**

(74) Representative: **Clements, Andrew Russell Niel et al**
**Schlich & Co**
**34 New Road**
**Littlehampton, West Sussex BN17 5AT (GB)**

(54) **Method for improving flavor of astaxanthin-containing extract**

(57) A method for improving the flavor of an astaxanthin-containing extract of the present invention includes the steps of: mixing 0.5 to 1000 parts by weight of ethanol and 1 part by weight of the astaxanthin-containing extract so as to obtain an ethanol mixture; collecting a solid precipitated from the ethanol mixture obtained; and drying the solid collected. In particular, the astaxanthin-containing extract, i.e., the starting material, is an extract from a green alga.

**Fig. 1**

Parts by weight of ethanol with respect to 1 part by weight of extract

**Description**

BACKGROUND OF THE INVENTION

1. Field of the Invention

The present invention relates to a method for improving the flavor of an astaxanthin-containing extract and to a flavor-improved astaxanthin-containing extract obtained by the method.

2. Description of the Related Art

Astaxanthin is a carotenoid imparting a red color, and is known to have a potent antioxidative effect. For this reason, it is used as a pigment in food, cosmetics, health food products, and pharmaceuticals. Some astaxanthins are chemically synthesized, and astaxanthins are also naturally occurring. Naturally-occurring astaxanthins are extracted, for example, from *Eucarida* such as euphausiids and *Pandalus borealis*, from *Phaffia* yeast, and from algae. However, astaxanthin cannot be produced efficiently from *Eucarida* such as euphausiids or from yeast because of their low astaxanthin content. On the other hand, algae are encysted as a result of a change in their external environment and accumulate astaxanthin within the algal cells. Thus, production of astaxanthin from algae has been investigated.

Since an astaxanthin molecule has two hydroxyl groups, astaxanthin often is present as a monoester or diester in which a fatty acid is esterified with one or both of the two hydroxyl groups. In particular, monoester forms are usually present in large quantities. The free form in which no fatty acid is bound to any hydroxyl group is present in very minute quantities. When compared with the free form, such ester forms generally have great stability against light, oxygen, and other similar conditions, are easy to handle during production, and may be absorbed readily into the body of animals fed with the astaxanthin.

However, fatty acid esters of xanthophylls such as astaxanthin require special consideration during their production because they are similar in various chemical behaviors to triglycerides and diglycerides, which are neutral lipids having ester bonds to fatty acids. In particular, xanthophyll-producing algae produce the above-mentioned neutral lipids in large quantities concurrently with the production of xanthophylls. Thus, if ordinary organic solvents are used to extract xanthophylls, neutral lipids will be extracted in combination with xanthophylls, and in larger quantities than the xanthophylls. In other words, neutral lipids containing xanthophylls at a low concentration (10 wt% or less) will be recovered. Therefore, in order to obtain a high concentration of astaxanthin, it is necessary to separate the fatty acid esters of astaxanthin from the neutral lipids, both of which have similar chemical behaviors. Moreover, these neutral lipids containing xanthophylls at a low concentration (10 wt% or less) have an odor or malodor coming from the raw material from which the xanthophyll is obtained, and elimination of such an odor is also important in broadening the range of applications for the astaxanthin product.

An example of a method for recovering xanthophylls from large quantities of fats and oils (neutral lipids) is a crystallization method. For example, Japanese Laid-Open Patent Publication No. 7-118226 discloses a method of purifying astaxanthin by crystallizing astaxanthin from a chloroform solution containing astaxanthin. Moreover, WO 97/23436 discloses a method of purifying xanthophylls by hydrolyzing an oil-containing component extracted from marigold flower petals to form a xanthophyll diester, then converting the xanthophyll diester into a xanthophyll in the free form, and crystallizing the xanthophyll in the free form. However, crystallization by using chloroform is not suitable for use in food production processes because of the toxicity of chloroform and other problems. Moreover, crystallization has the following problems: the concentration of a substance desired to be crystallized has to be not less than a specific concentration; substances other than the desired substance may crystallize out; the purity of the crystallized product is low when the target substance is not likely to form a crystalline structure; and so on. Furthermore, both of the above-described methods crystallize the free form of xanthophylls, and are incapable of providing xanthophylls in the form of a mixture of xanthophyll fatty acid esters.

There are disclosed methods for recovering a precipitated fatty acid ester of lutein, which is a carotenoid, as an ester mixture (Japanese Laid-Open Patent Publication No. 2002-30068, U.S. Patent No. 4048203, and WO 99/54408). In Japanese Laid-Open Patent Publication No. 2002-30068, a lutein fatty acid ester derived from a plant is purified by dissolving in warm acetone, removing the precipitate from the acetone solution, distilling off the acetone, dissolving the obtained residue in warm propanol and/or warm butanol, and cooling for precipitation. In U.S. Patent No. 4048203, a marigold flower extract is dissolved in a warm alcohol, the solution is filtered to remove insolubles, the filtrate is then cooled to obtain a lutein fatty acid ester as a precipitate. In WO 99/54408, a xanthophyll ester extracted with a hydrocarbon solvent is mixed with an alcohol to dissolve impurities, the dissolved impurities are removed, and then, a trans-xanthophyll ester is obtained. As described above, there are various methods for the purification of lutein fatty acid esters, but it is unclear whether or not the odor coming from a raw material is sufficiently removed.

Methods for the removal from carotenoid pigments of the odor derived from the raw material have been investigated. For example, in Japanese Laid-Open Patent Publication No. 9-48927, a material containing a carotenoid pigment is treated by mixing with a water-soluble organic solvent and then with a fat-soluble organic solvent to give a solid as a carotenoid pigment. However, in view of its use for food products and the like, it is not favorable to use fat-soluble organic solvents. Moreover, in Japanese Laid-Open Patent Publication Nos. 7-304977, 7-304978, and 7-304979, a material containing a carotenoid pigment is hydrolyzed, washed with an organic solvent (e.g., ethanol or supercritical carbon dioxide) in which carotenoids are very insoluble. These methods are the methods for the recovery of carotenoids in the free form rather than the methods for the recovery of carotenoids remaining in the form of an ester mixture.

## SUMMARY OF THE INVENTION

It is an object of the present invention to provide a method for improving the flavor of an astaxanthin-containing extract and to provide a flavor-improved astaxanthin-containing extract.

The present invention provides a method for improving the flavor of an astaxanthin-containing extract comprising the steps of:

mixing 0.5 to 1000 parts by weight of ethanol and 1 part by weight of the astaxanthin-containing extract so as to obtain an ethanol mixture;
collecting a solid from the ethanol mixture obtained; and
drying the solid collected.

In one embodiment, the astaxanthin-containing extract is an extract from a green alga.

In a further embodiment, the green alga is a unicellular alga belonging to the genus *Haematococcus.*

In one embodiment, the amount of ethanol added in the mixing step is preferably 1 to 100 parts by weight, more preferably 2 to 50 parts by weight, further preferably 4 to 30 parts by weight, to 1 part by weight of the astaxanthin-containing extract.

In an embodiment, the purity of ethanol added in the mixing step is 99% (v/v) or higher.

In one embodiment, the temperature of the ethanol mixture in the collecting step is 10°C or lower.

In an embodiment, the mixing step is performed at a temperature of 60°C or lower, preferably at a temperature between 5°C and 50°C.

The present invention also provides a flavor-improved astaxanthin-containing extract obtained by any of the above-described method.

According to the present invention, an astaxanthin-containing extract having improved flavor as a result of the removal of odor or malodor derived from the raw material containing astaxanthin can be obtained by a very simple process. Therefore, the flavor-improved astaxanthin-containing extract of the present invention can be preferably used as an ingredient of food products and pharmaceuticals, i.e., applications in which flavors are a matter of concern.

## BRIEF DESCRIPTION OF THE DRAWINGS

Fig. 1 is a graph showing the relationship between the amounts of ethanol used and the concentration of astaxanthin in the dry astaxanthin products obtained.
Fig. 2 is a gas chromatogram of the odorous component of a *Haematococcus* alga extract (untreated product) obtained in Reference Example 1.
Fig. 3 is gas chromatogram of the odorous component of a flavor-improved product treated with 98% (v/v) ethanol obtained in Example 6.
Fig. 4 is a gas chromatogram of the odorous component of a flavor-improved product treated with 99% (v/v) ethanol obtained in Example 6.

## DESCRIPTION OF THE PREFERRED EMBODIMENTS

Astaxanthin-Containing Extract

In the present invention, the term "astaxanthin" means at least one compound selected from the group consisting of the free form of astaxanthin, astaxanthin fatty acid monoesters, and astaxanthin fatty acid diesters. Examples of fatty acids in astaxanthin fatty acid esters include lauric acid, myristic acid, pentadecanoic acid, palmitic acid, palmitoleic acid, heptadecanoic acid, elaidic acid, ricinoleic acid, petroselinic acid, vaccenic acid, eleostearic acid, punicic acid, licanic acid, parinaric acid, gadoleic acid, 5-eicosenoic acid, 5-docosenoic acid, cetoleic acid, erucic acid, 5,13-docosaclienoic acid, selacholeic acid, decenoic acid, dodecenoic acid, oleic acid, stearic acid, eicosapentaenoic acid, docosahexaenoic acid, linoleic acid, α-linolenic acid, and arachidonic acid.

In the present invention, the term "astaxanthin-containing extract" refers to an astaxanthin-containing oily substance extracted from a naturally occurring product. The oily substance may be a viscous liquid as well as a solid. The oily substance may be an oily substance that has been subjected to only extraction (i.e., other than extraction, neither concentration nor purification of astaxanthin has been performed), or may be an oily substance that has already been subjected to concentration or purification. Examples of the naturally-occurring products include red yeast; the shell of crustaceans such as *Tigriopus* (red water flea) and krills; and microalgae such as green algae, all of which contain astaxanthin. In the present invention, an extract from a green alga is preferably used.

There is no particular limitation on the green alga as long as the green alga has an ability to produce astaxanthin. For example, unicellular algae belonging to the genus *Haematococcus* are preferably used. Examples of preferred green algae include *Haematococcus pluvialis (H. pluvialis)*, *Haematococcus lacustris (H. lacustris)*, *Haematococcus capensis (H. capensis)*, *Haematococcus droebakensi (H. droebakensi)*, and *Haematococcus zimbabwiensis* (*H. zimbabwiensis*).

For example, an astaxanthin-containing extract from a green alga is produced in the following manner. First, an encysted green alga is cultivated in a nutrient medium with a supply of carbon dioxide under irradiation with light at a photosynthetically active photon flux input of, for example, 8000 μmol-photon/m$^3$/s or more, and then an astaxanthin-containing oily substance is extracted. There is no particular limitation on the means for extracting the astaxanthin-containing oily substance, and a means commonly used by those skilled in the art is employed. For example, the astaxanthin-containing oily substance is extracted from the cultivated green alga by extraction with a solvent, mechanical breaking (e.g., with a bead beater), squeezing, or a combination thereof. As the solvent, it is possible to use an organic solvent such as chloroform, hexane, acetone, methanol, or ethanol. Alternatively, the oily substance may be extracted by supercritical extraction. In the case where a solvent is used in extraction, the solvent is removed after the extraction by a means commonly used by those skilled in the art.

Algae such as *Haematococcus,* accumulate astaxanthin, which is a xanthophyll, within algal cells, but simultaneously produce oily substances such as neutral lipids. Thus, with a common extraction method for the extraction of astaxanthin, astaxanthin is extracted together with the neutral lipids. Although the concentration of astaxanthin in oily substances derived from algae varies depending on the extraction solvent or the extraction method, the astaxanthin concentration is generally about 1 to 10 wt%. It should be noted that depending on the culture conditions, astaxanthin in algae is mainly composed of astaxanthin fatty acid monoesters, which account for at least 50 wt%, more preferably 60 wt% or more, and even more preferably 70 wt% or more of the entire xanthophyll content. Astaxanthin fatty acid diesters are contained in an amount of about 15 to 30 wt%, and the free form of astaxanthin is often 1 wt% or less. In the present invention, an astaxanthin-containing oily substance derived from an alga belonging to the genus *Haematococcus* of green algae, preferably *Haematococcus pluvialis*, is preferably used as the astaxanthin-containing extract.

Method for Improving the Flavor of an Astaxanthin-Containing Extract

The method for improving a flavor of an astaxanthin-containing extract of the present invention includes the steps of:

mixing 0.5 to 1000 parts by weight of ethanol and 1 part by weight of the astaxanthin-containing extract so as to obtain an ethanol mixture;
collecting a solid precipitated from the ethanol mixture obtained; and
drying the solid collected.

In the present invention, based on the comparatively low solubility of astaxanthin in ethanol, an ethanol-soluble component having a characteristic odor derived from the raw material from which the astaxanthin-containing extract is obtained is removed, and an ethanol-insoluble component containing the astaxanthin is recovered.

In the flavor improving method of the present invention, an astaxanthin-containing extract is first mixed with ethanol. The astaxanthin-containing extract used is an astaxanthin-containing oily substance extracted from a naturally-occurring product as described above.

The greater the amount of ethanol used in mixing, the higher the purity of astaxanthin and the weaker the characteristic odor of the astaxanthin-containing extract, resulting in an enhanced flavor improving effect. On the other hand, the greater the amount of ethanol used, the lower the yield of astaxanthin. Therefore, the amount of ethanol used is 0.5 to 1000 parts by weight, preferably 1 to 100 parts by weight, more preferably 2 to 50 parts by weight, even more preferably 4 to 30 parts by weight, and still more preferably 6 to 20 parts by weight to 1 part by weight of the astaxanthin-containing extract.

Moreover, the higher the purity of ethanol used in mixing, the higher the purity of the collected astaxanthin and the weaker the characteristic odor of the astaxanthin-containing extract, resulting in an enhanced flavor improving effect.

Therefore, the purity of ethanol used in the present invention is preferably 98% (v/v) or higher, more preferably 99% (v/v) or higher, and even more preferably 99.5% (v/v) or higher.

There is no particular limitation on the mixing means. For example, mixing is performed by stirring, shaking, and so on. Since the purpose of this mixing step is to dissolve in ethanol any substance contained in the astaxanthin-containing extract that is readily soluble in ethanol, mixing or stirring does not have to be performed to a particularly great extent, and for example, gentle stirring is sufficient for this purpose. Mixing to an extent similar to that of mixing commonly performed by those skilled in the art, e.g., stirring with a stirring blade or a magnetic stirrer, mixing by ultrasonic irradiation, or shaking with a shaker, is sufficient. The mixing step is performed at a temperature sufficiently high so that the viscosity of the astaxanthin-containing extract is reduced so as to facilitate mixing with ethanol, but at a temperature not so high that oils and fats in the astaxanthin-containing extract may be degraded, resulting in deterioration of the flavor as well as a reduction in the yield of astaxanthin. Mixing is preferably performed at a temperature of 60°C or lower and more preferably between 5°C and 50°C.

In the next step, the solid is precipitated and collected from the ethanol mixture. The temperature of the ethanol mixture at the time of precipitation and collection is preferably room temperature or lower, more preferably 10°C or lower, and even more preferably 5°C or lower. The ethanol mixture may also be allowed to stand so as to facilitate the collection of the solid. There is no particular limitation on the standing time. For example, in the case where the ethanol mixture is allowed to stand at 5°C, the solid is precipitated within about 1 to 48 hours.

The precipitated solid is collected by, for example, filtration, centrifugation, or decantation. Then, residual ethanol is removed from the collected solid by drying under reduced pressure or by, for example, blowing an inert gas such as nitrogen into the collected solid.

Flavor-Improved Astaxanthin-Containing Extract

The flavor-improved astaxanthin-containing extract obtained in the above-described manner contains astaxanthin usually in an amount of at least 10 wt%, preferably at least 15 wt%, more preferably at least 20 wt%, and even more preferably at least 25 wt% in terms of the free form, although the astaxanthin content varies depending on the concentration of astaxanthin in the astaxanthin-containing extract, i.e., the starting material. The astaxanthin in the flavor-improved extract is in the form of free astaxanthin, astaxanthin fatty acid monoester, astaxanthin fatty acid diester, or a mixture thereof depending on the astaxanthin contained in the raw material of the astaxanthin-containing extract. For example, in the case where the raw material is a *Haematococcus* alga, more than half of astaxanthin may be fatty acid monoesters.

Moreover, in the flavor-improved astaxanthin-containing extract, the characteristic odor derived from the raw material is reduced to such an extent that the odor is only slightly perceptible or nearly or completely unperceptible.

Examples

Hereinafter, the present invention will be described by means of examples in which the *Haematococcus pluvialis* K0084 strain is used. However, the present invention is not limited to these examples. It should be noted that in the examples, the measurement of astaxanthin concentration and the sensory evaluation of flavor were performed according to the following methods.

(Method for Measurement of Astaxanthin Concentration)

The extract was diluted with dimethyl sulfoxide (DMSO) as appropriate, and then the concentration of astaxanthin (the concentration of astaxanthin fatty acid esters in terms of free form of astaxanthin) in an extract was calculated using the following equation:

$$\text{Astaxanthin concentration (wt\%)} = (A \times 100 \times F) / (W \times 2085)$$

where A represents the absorbance (optical path length: 1 cm) at 478 nm of a sample, F represents the dilution factor of the sample, and W represents the weight (g) of the sample.

(Method for Sensory Evaluation)

An odor characteristic of *Haematococcus* alga in the obtained sample was evaluated according to the criteria below. The evaluation was performed by an evaluator who had passed a gustatory test (a five-basic taste discrimination test: see "New edition: Gustatory test handbook", 1973, edited by Union of Japanese Scientists and Engineers, published by JUSE Press Ltd.) and an olfactory test (T&T olfactometer method by using Odor Standard for Selecting Panel manufactured by Daiichi Yakuhin Sangyo Ltd.) and was able to perform a proper sensory evaluation.

*Criteria for flavor evaluation*

1: The specific odor is completely unperceptible.
2: The specific odor is nearly unperceptible.
3: The specific odor is slightly perceptible.
4: The specific odor is strongly perceptible.
5: The specific odor is very strongly perceptible.

(Reference Example 1: Preparation of a *Haematococcus* alga extract)

*Haematococcus pluvialis* K0084 strain was inoculated into a medium (a low nutrient medium) containing the components shown in Table 1 below.

Table 1

| Components | g/L |
| --- | --- |
| $KNO_3$ | 0.41 |
| $K_2HPO_4$ | 0.04 |
| $MgSO_4 \cdot 7H_2O$ | 0.075 |
| $CaCl_2 \cdot 2H_2O$ | 0.036 |
| Citric acid (anhydrous) | 0.006 |
| Ammonium iron (III) citrate | 0.006 |
| $EDTA \cdot 2Na$ | 0.001 |
| $Na_2CO_3$ | 0.02 |
| $CuSO_4 \cdot 5H_2O$ | 0.00286 |
| $H_3BO_4$ | 0.00181 |
| $MnCl_2 \cdot 4H_2O$ | 0.00022 |
| $ZnSO_4 \cdot 7H_2O$ | 0.00008 |
| $Na_2MoO_4$ | 0.000021 |
| $Co(NO_3)_2 \cdot 6H_2O$ | 0.000000494 |

More specifically, 1 L of the low nutrient medium was placed in a 1.5 L flat culture flask, and the encysted K0084 strains were inoculated into the medium. The K0084 strains were cultivated for 7 days at 25°C under irradiation with light at a photosynthetically active photon flux input of 25000 $\mu$mol-p/m$^3$/s using a white fluorescent lamp, while bubbling a gas containing 3 vol% of $CO_2$ into the medium at a rate of 0.5 L/minute (i.e., at an aeration rate of 0.5 vvm). After 7 days, the encysted K0084 strains were collected, and adjusted so that the concentration of the encysted K0084 strains in the low nutrient medium was $1.5 \times 10^6$ cells/mL.

Then, 9 L of the low nutrient medium were placed in a flat culture tank in which acrylic transparent plates are opposite to each other so that the distance between the inner walls of the culture tank was 3 cm, and 1 L of the above-described encysted K0084 strains were inoculated in the medium so that the concentration was $1.5 \times 10^5$ cells/mL to start cultivation. The encysted K0084 strains were cultivated for 21 days at 25°C under irradiation with light at a photosynthetically active photon flux input of 25000 $\mu$mol-p/m$^3$/s using six white fluorescent lamps provided on each side of the flat culture tank while bubbling a gas containing 3 vol% of $CO_2$ into the medium at a rate of 5 L/minute (i.e., at an aeration rate of 0.5 vvm). The culture suspension was removed, and algal cells in the suspension were washed with water and taken into a tube designed for exclusive use with a bead beater. After adding zirconia beads into this tube, 50 mL of ethanol was added thereto, and the algal cells were beaten with the bead beater. The beaten product was separated into a supernatant and a precipitate by a centrifugation, and the supernatant was collected. Another 50 mL of ethanol was added to the precipitate, and the same operation as described above was repeated until the color of the precipitate became almost completely white. The collected ethanol fractions were combined, and ethanol was removed under reduced pressure to obtain a green alga extract. The concentration of astaxanthin in this extract was 13.1 wt% in terms of the free form.

(Example 1: Preparation of a flavor-improved astaxanthin-containing extract - 1)

First, 30 g of the *Haematococcus* alga extract (astaxanthin content in terms of free form: 13.1 wt%) obtained in Reference Example 1 above was warmed to 50°C. Then, 240 g of ethanol (purity: 99.5% (v/v) or higher) was added thereto, and the mixture was sufficiently mixed by stirring with a magnetic stirrer. After the resultant solution was allowed to stand overnight at 5°C, a precipitated solid was collected by filtration with a filter paper. The collected solid was concentrated under reduced pressure to remove the ethanol. Thus, 6.8 g of a dry product having an astaxanthin content of 31.5 wt% in terms of free form was obtained (yield: 54%).

(Example 2: Preparation of a flavor-improved astaxanthin-containing extract - 2)

The same procedure as in Example 1 was performed except that the standing temperature of the solution of the *Haematococcus* alga extract in ethanol as in Example 1 was room temperature. Thus, 5.0 g of a dry product having an astaxanthin content of 34.8 wt% in terms of free form was obtained (yield: 44%).

(Example 3: Flavor evaluation of the flavor-improved astaxanthin-containing extracts)

Each sample of the *Haematococcus* alga extract obtained in Reference Example 1 and the flavor-improved astaxanthin-containing extracts obtained in Examples 1 and 2 was subjected to a sensory evaluation of the odor characteristic of the *Haematococcus* alga. The results are shown in Table 2.

Table 2

| Sample | Evaluation of flavor |
|---|---|
| Haematococcus alga extract | 5 |
| Dry product of Example 1 | 2 |
| Dry product of Example 2 | 3 |

Although the *Haematococcus* alga extract had a strongly perceptible characteristic odor, the characteristic odor was reduced to an only slightly perceptible extent or a nearly unperceptible extent by the ethanol treatment.

(Example 4: Examination of the amount of ethanol used)

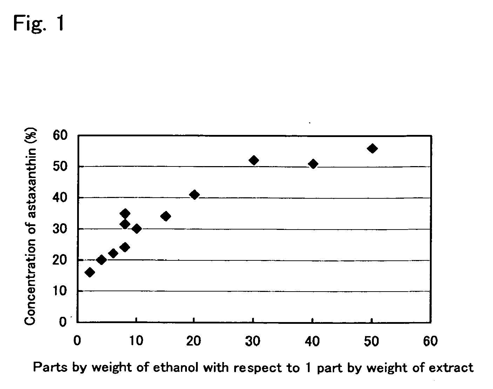

First, various amounts ranging from 2 to 50 parts by weight of ethanol (purity: 99.5% (v/v) or higher) were added to 1 part by weight of the *Haematococcus* alga extract obtained in Reference Example 1 at room temperature, and each mixture was sufficiently mixed by stirring the mixture with a stirring blade. The resultant solution was allowed to stand overnight at a temperature in the range from 5 to 10°C, and a precipitated solid was collected by filtration with a filter paper. The collected solid was concentrated under reduced pressure to remove ethanol, and thus a dry product was obtained. The obtained dry products were subjected to the measurement of astaxanthin concentration and the sensory evaluation of the flavor characteristic of the *Haematococcus* alga. Fig. 1 shows the relationship between the amounts of ethanol used and the concentration of astaxanthin in the obtained dry products. It should be noted that Fig. 1 also shows the results for the dry products obtained in Examples 1 and 2. Moreover, Table 3 shows the results of the sensory evaluation of the flavor of the dry products obtained in Example 4 in conjunction with the concentration and the yield of astaxanthin.

Table 3

| Amount of ethanol used (parts by weight) | Astaxanthin in the obtained dry product | | |
|---|---|---|---|
| | Evaluation of flavor | Concentration (wt%) (in terms of free form) | Yield (%) |
| 2 | 4 | 16 | 76 |
| 4 | 3 | 20 | 72 |
| 6 | 2 | 22 | 66 |
| 8 | 2 | 24 | 62 |
| 10 | 2 | 30 | 49 |
| 15 | 2 | 34 | 43 |
| 20 | 1 | 41 | 41 |
| 30 | 1 | 52 | 32 |
| 40 | 1 | 51 | 15 |
| 50 | 1 | 56 | 16 |

It was found that the concentration of astaxanthin in the dry products increases as the added amount of ethanol increases, and the flavor also tends to be improved with the increase in the astaxanthin concentration. On the other hand, it was found that the yield of astaxanthin tends to decrease as the added amount of ethanol used increases.

(Example 5: Examination of the temperature in the mixing step)

Six mixtures, each containing 10 parts by weight of ethanol (purity: 99.5% (v/v) or higher) added to 1 part by weight of the *Haematococcus* alga extract obtained in Reference Example 1, were prepared and were sufficiently mixed by stirring with a stirring blade at the various temperatures (10 to 60°C) shown in Table 4. After each of the resultant solutions was allowed to stand overnight at 10°C, a precipitated solid was collected by filtration with a filter paper. The collected solid was concentrated under reduced pressure to remove ethanol, and thus a dry product was obtained. The obtained dry products were subjected to the measurement of astaxanthin concentration and the sensory evaluation of the flavor characteristic of the *Haematococcus* alga. The results are shown in Table 4. It should be noted that in the case where the mixture was mixed at 60°C and allowed to stand overnight, only a little amount of solid was visually observed, and so the mixture was allowed to stand for two nights.

Table 4

| Mixing temperature | Astaxanthin in the obtained dry product | | |
|---|---|---|---|
| | Evaluation of flavor | Concentration (wt%) (in terms of free form) | Yield (%) |
| 10°C | 2 | 31 | 52 |
| 15°C | 3 | 31 | 39 |
| Room temperature (22°C) | 3 | 28 | 49 |
| 30°C | 3 | 27 | 39 |
| 40°C | 2 | 29 | 50 |
| 60°C, allowed to stand for two nights | 3 | 28 | 46 |

It was found that there is not much difference in flavor and astaxanthin concentration among the obtained dry products as long as the mixing temperature is not higher than 60°C.

(Example 6: Examination of the purity of ethanol)

Three mixtures, each containing 10 parts by weight of ethanol having various purities as shown in Table 5 added to 1 part by weight of the *Haematococcus* alga extract obtained in Reference Example 1 at room temperature, were prepared and each mixture was sufficiently mixed by stirring with a stirring blade. After each resultant solution was allowed to stand overnight at 10°C, a precipitated solid was collected by filtration with a filter paper. The collected solid was concentrated under reduced pressure to remove ethanol and water, and thus a dry product was obtained. The obtained dry products were subjected to the measurement of astaxanthin concentration and the sensory evaluation of the flavor characteristic of the *Haematococcus* alga. The results are shown in Table 5.

Table 5

| Purity of ethanol | Astaxanthin in the obtained dry product | | |
|---|---|---|---|
| | Evaluation of flavor | Concentration (wt%) (in terms of free form) | Yield (%) |
| 99.5%(v/v) or higher | 2 | 27 | 60 |
| 99%(v/v) | 2 | 22 | 75 |
| 98%(v/v) | 4 | 21 | 69 |

It was found that the concentration of astaxanthin in the dry products increases as the purity of ethanol used increases, and the flavor is also improved with the increase in the astaxanthin concentration.

(Example 7: Examination of solvents other than ethanol)

A mixture containing 10 parts by weight of ethanol (purity: 99.5% (v/v) or higher) added to 1 part by weight of the *Haematococcus* alga extract obtained in Reference Example 1 was prepared. A second mixture was prepared in the same manner except that 10 parts of 2-propanol were used instead of the ethanol. Each mixture was sufficiently mixed by stirring with a stirring blade. After each resultant solution was allowed to stand for 3 hours at 5°C, the solution was sufficiently stirred again. This solution was poured all at once into a Buchner funnel in which a filter paper (quantitative filter paper φ 90 mm, No. 5C; manufactured by Advantec Toyo Kaisha, Ltd.) was placed, and a precipitated solid was collected by filtration under reduced pressure. At this time, the amount of time required to complete the filtration was measured. Then, the collected solid was concentrated under reduced pressure to remove the ethanol or 2-propanol, and thus a dry product was obtained. The obtained dry products were subjected to the measurement of astaxanthin concentration and the sensory evaluation of the flavor characteristic of the *Haematococcus* alga. The results are shown in Table 6.

Table 6

| Solvent used | Filtration time | Astaxanthin in the obtained dry product | | |
|---|---|---|---|---|
| | | Evaluation of flavor | Concentration (wt%) (in terms of free form) | Yield (%) |
| Ethanol | 6 minutes 6 seconds | 2 | 27 | 60 |
| 2-Propanol | 57 minutes 22 seconds | 3 | 33 | 52 |

In the case where 2-propanol was used, a dry product with a high astaxanthin concentration and an improved flavor could be obtained. However, clogging of pores of the filter paper occurred, and the filtration required a very long period of time. Therefore, it was found that solvents other than ethanol are not suitable for practical industrial use.

(Example 8: Measurement of an odorous substance, 2,4-heptadienal)

The characteristic odor coming from a raw material containing astaxanthin is derived from a plurality of odorous substances. One of the causative substances is 2,4-heptadienal. 2,4-Heptadienal is thought to be one of the substances that cause the foul odor of tap water (see Toshiyuki Kataoka, the summaries of the 47-th JWWA Research Conference and Symposium held by the Japan Water Works Association, 1996, pp. 472-473). For this reason, 2,4-heptadienal in the *Haematococcus* alga extract (untreated product) obtained in Reference Example 1 and the dry products (flavor-improved products) treated with ethanol of a purity of 98% (v/v) and 99% (v/v) obtained in Example 6 was measured.
First, 0.5 g each of the samples was placed into a gastight vial, and ventilated with nitrogen to a headspace of the vial while keeping the samples warm at 60°C to collect a gas discharged from the vial. Then, 1 L of the collected gas was passed through an adsorbent (NeedlEx; manufactured by Shinwa Chemical Industries Ltd.) to adsorb the odorous component onto the adsorbent. The adsorbed odorous component was analyzed with a gas chromatograph (GC) equipped with a flame ionization detector (FID). The conditions of the analysis were as follows:

(GC)

Column: ZB-1 (60 m $\times$ 0.32 mm I.D. df = 1.0 $\mu$m)
Column temperature: 40°C (2 minutes) $\rightarrow$ (10°C/minute) $\rightarrow$ 300°C (5 minutes)
Inlet temperature: 250°C
Carrier gas (He) pressure: 121.4 kPa

(FID)

Detector temperature: 300°C
Makeup gas (He) flow rate: 30.0 mL/minute

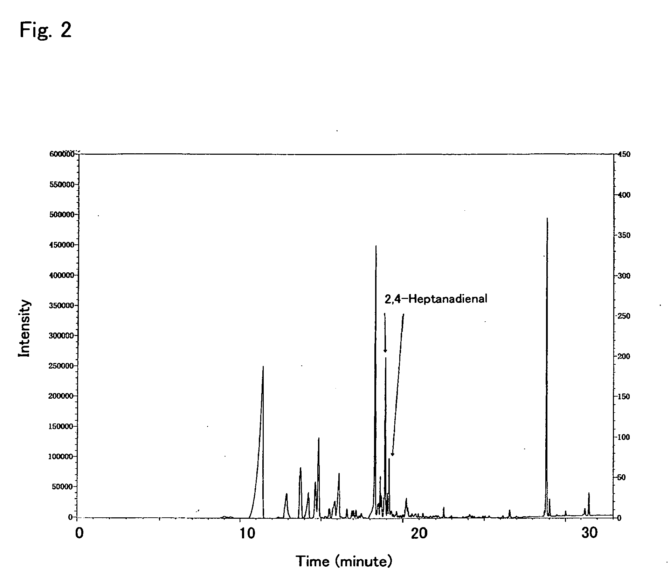

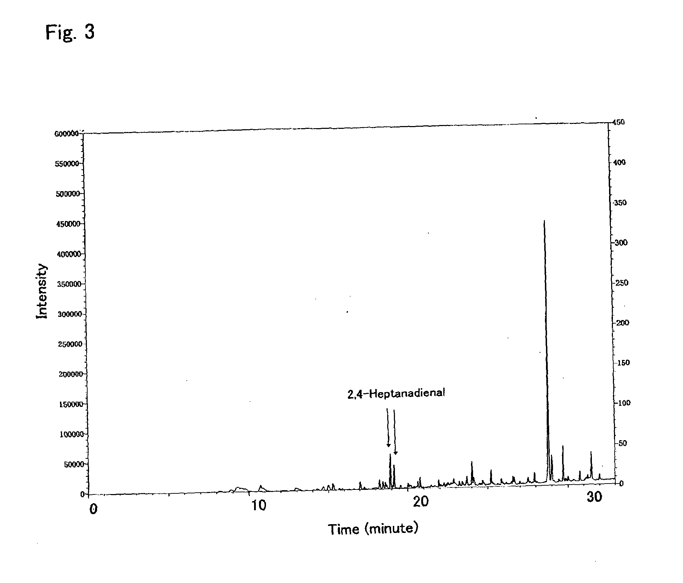

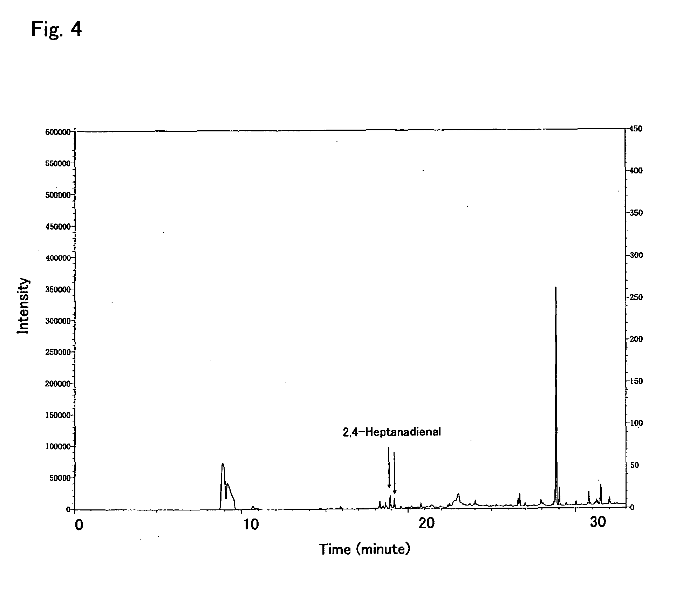

Figs. 2 to 4 show respective gas chromatograms. As can be seen from these gas chromatograms, 2,4-heptadienal in the flavor-improved products considerably decreased when compared with that in the untreated product. The results were in good agreement with the results of the sensory evaluation.

According to the method of the present invention, the flavor of an astaxanthin-containing extract having a characteristic odor derived from a raw material can be improved. Therefore, the resultant flavor-improved astaxanthin-containing extract can be suitably used in the field of food products, pharmaceuticals, cosmetics, etc.

The invention may be embodied in other forms without departing from the spirit or essential specifics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

**Claims**

1.   A method for improving flavor of an astaxanthin-containing extract comprising the steps of:

mixing 0.5 to 1000 parts by weight of ethanol and 1 part by weight of the astaxanthin-containing extract so as to obtain an ethanol mixture;
collecting a solid from the ethanol mixture obtained; and
drying the solid collected.

2.   The method of claim 1, wherein the astaxanthin-containing extract is an extract from a green alga.

3.   The method of claim 2, wherein the green alga is a unicellular alga belonging to the genus *Haematococcus.*

4.   The method of any one of claims 1 to 3, wherein the amount of ethanol added in the mixing step is 1 to 100 parts by weight.

5.   The method of claim 4, wherein the amount of ethanol added in the mixing step is 2 to 50 parts by weight.

6.   The method of claim 5, wherein the amount of ethanol added in the mixing step is 4 to 30 parts by weight.

7.   The method of any one of claims 1 to 6, wherein the purity of ethanol added in the mixing step is 99% (v/v) or higher.

8.   The method of any one of claims 1 to 7, wherein the temperature of the ethanol mixture in the collecting step is 10°C or lower.

9.   The method of any one of claims 1 to 8, wherein the mixing step is performed at a temperature of 60°C or lower.

10.   The method of claim 9, wherein the mixing step is performed at a temperature between 5°C and 50°C.

11.   A flavor-improved astaxanthin-containing extract obtained by the method of any one of claims 1 to 10.

Fig. 1

Parts by weight of ethanol with respect to 1 part by weight of extract

Fig. 2

Fig. 3

Fig. 4

Europäisches
Patentamt

European
Patent Office

Office européen
des brevets

# EUROPEAN SEARCH REPORT

Application Number

EP 09 00 1218

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | DATABASE WPI Week 199717<br>Thomson Scientific, London, GB; AN<br>1997-188448<br>XP002526252<br>& JP 09 048927 A (SANEIGEN FFI KK)<br>18 February 1997 (1997-02-18)<br>* abstract * | 1-11 | INV.<br>A23L1/275<br>C12P23/00<br>A61K8/97<br>A61K31/122<br>A61K36/05 |
| D,X | DATABASE WPI Week 199604<br>Thomson Scientific, London, GB; AN<br>1996-036020<br>XP002526253<br>& JP 07 304978 A (SANEIGEN FFI KK)<br>21 November 1995 (1995-11-21)<br>* abstract * | 1-11 | |
| X | CA 2 647 451 A1 (NIPPON OIL CORP [JP];<br>ASAHI KASEI PHARMA CORP [JP])<br>11 October 2007 (2007-10-11)<br>* page 22, lines 1-8; claims *<br>* page 4, line 7 - line 11 * | 1-11 | |
| X | EP 1 800 674 A (YAMAHA MOTOR CO LTD [JP])<br>27 June 2007 (2007-06-27)<br>* page 5, line 45 - line 57 * | 11 | TECHNICAL FIELDS SEARCHED (IPC)<br><br>A23L<br>C12P<br>A61K |
| X | WO 01/83437 A (HAITAI CONFECTIONERY CO LTD [KR]; HAN JI YOUNG [KR]; LEE SEUNG JAE [KR] 8 November 2001 (2001-11-08)<br>* example 1 * | 11 | |

The present search report has been drawn up for all claims

| Place of search | Date of completion of the search | Examiner |
|---|---|---|
| Munich | 30 April 2009 | Smeets, Dieter |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 09 00 1218

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2009

| Patent document cited in search report | | Publication date | Patent family member(s) | | Publication date |
|---|---|---|---|---|---|
| JP 9048927 | A | 18-02-1997 | NONE | | |
| JP 7304978 | A | 21-11-1995 | NONE | | |
| CA 2647451 | A1 | 11-10-2007 | AU | 2007232749 A1 | 11-10-2007 |
| | | | EP | 2017262 A1 | 21-01-2009 |
| | | | WO | 2007114461 A1 | 11-10-2007 |
| | | | KR | 20080112338 A | 24-12-2008 |
| EP 1800674 | A | 27-06-2007 | US | 2007135521 A1 | 14-06-2007 |
| WO 0183437 | A | 08-11-2001 | AU | 5680701 A | 12-11-2001 |
| | | | EP | 1278725 A1 | 29-01-2003 |
| | | | JP | 2003531888 T | 28-10-2003 |
| | | | KR | 20000053886 A | 05-09-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7118226 A
- WO 9723436 A
- JP 2002030068 A
- US 4048203 A
- WO 9954408 A

- JP 9048927 A
- JP 7304977 A
- JP 7304978 A
- JP 7304979 A

**Non-patent literature cited in the description**

- New edition: Gustatory test handbook. JUSE Press Ltd, 1973

- Toshiyuki Kataoka, the summaries of the 47-th JWWA Research Conference and Symposium. Japan Water Works Association, 1996, 472-473